# FASCICULE DE BREVET EUROPEEN

(11) **EP 0 656 364 B1**
(45) Date de publication et mention de la délivrance du brevet: **22.10.1997**
(21) Numéro de dépôt: 94402759.8
(22) Date de dépôt: 02.12.1994
(51) Int. Cl.: C07H 17/08, A61K 31/70

(54) **Nouveaux dérivés de l'érythromycine, leur procédé de préparation et leur application comme médicaments**
Erythromycinderivate, Verfahren zu ihrer Herstellung und ihre Verwendung als Arzneimittel
Erythromycin derivatives, their process of preparation and their use as medicaments

(30) Priorité: 03.12.1993 FR 9314505
(43) Date de publication de la demande: 07.06.1995
(73) Titulaire: ROUSSEL UCLAF, 93230 Romainville (FR)
(72) Inventeur: Agouridas, Constantin, F-94130 Nogent Sur Marne (FR); Chantot, Jean-Francois, F-94130 Nogent Sur Marne (FR); Tessot, Nicole, F-77410 CLaye-Souilly (FR)
(74) Mandataire: Vieillefosse, Jean-Claude

(56) Documents cités:
- EP-A- 0 487 411
- WO-A-90/11288
- JOURNAL OF ORGANIC CHEMISTRY., vol.57, no.16, 1992, EASTON US pages 4361 - 4367 A.B.JONES 'New Macrolide Antibiotics: Synthesis of a 14-Membered Azalide.'
- ANTIMICROBIAL AGENTS AND CHEMOTHERAPY, vol.35, no.5, 1991, WASHINGTON DC, USA. pages 922 - 928 D.J.HARDY ET AL. 'In Vitro Activity and In Vivo Efficacy of a New Series of 9-Deoxo-12-Deoxy-9,12-Epoxyerythromycin A Derivatives.'

## Description

La présente invention concerne de nouveaux dérivés de l'érythromycine, leur procédé de préparation et leur application comme médicaments. On connaissait déjà des dérivés de l'érythromycine 9-déoxo-9,12-epoxy (cf WO A 9011288) présentant des propriétés antibiotiques.

L'invention a pour objet les composés de formule (I) : 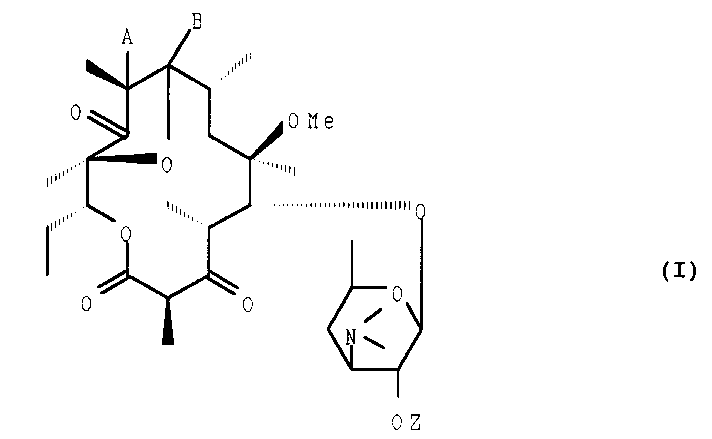 dans lesquels :
- ou bien A représente un atome d'hydrogène et B un radical OH,
- ou bien A et B forment ensemble une double liaison carbone-carbone,
et Z représente un atome d'hydrogène ou le reste d'un acide carboxylique.

L'invention a plus particulièrement pour objet les composés de formule (I) dans lesquels Z représente un atome d'hydrogène, ainsi que ceux dans lesquels A et B forment ensemble une double liaison carbone-carbone.

L'invention a plus particulièrement pour objet le composé de l'exemple 2.

Les produits de formule générale (I) possèdent une bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et, notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppurations pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits préférés de formule (I) définis précédemment et notamment le produit de l'exemple 2.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'homme, avec le produit décrit à l'exemple 2.

L'invention a également pour objet un procédé de préparation des composés de formule (I) caractérisé en ce que l'on soumet un composé de formule (II) : 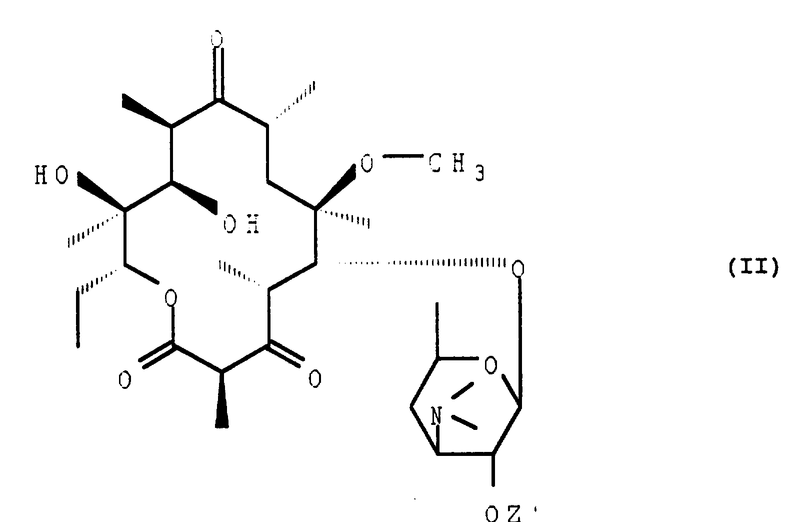 dans laquelle Z' représente le reste d'un acide carboxylique à l'action d'un agent capable d'introduire un pont époxyde en 9(12) et d'oxyder simultanément la fonction hydroxy en 11, pour obtenir le composé de formule (I_{A}) : 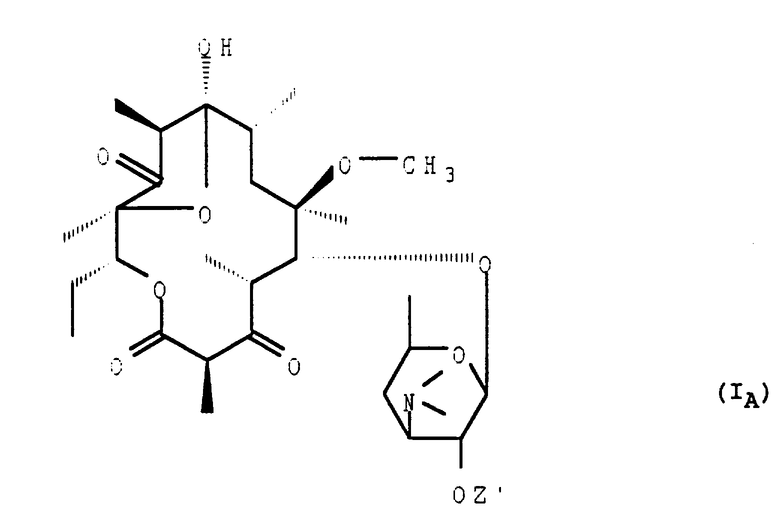 que l'on soumet si désiré :
- soit à l'action d'un agent de libération de la fonction hydroxyle en 2' pour obtenir le composé (I_{B}) : 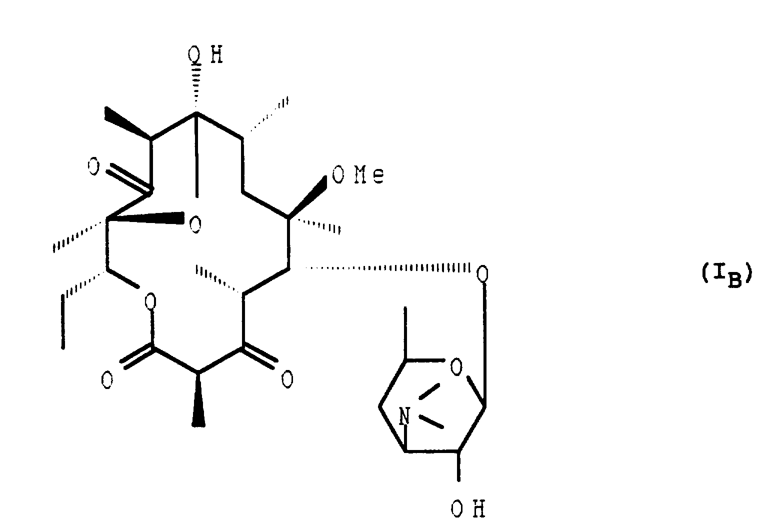
- soit à l'action d'un agent de déshydratation pour obtenir le composé de formule (I_{C}) : 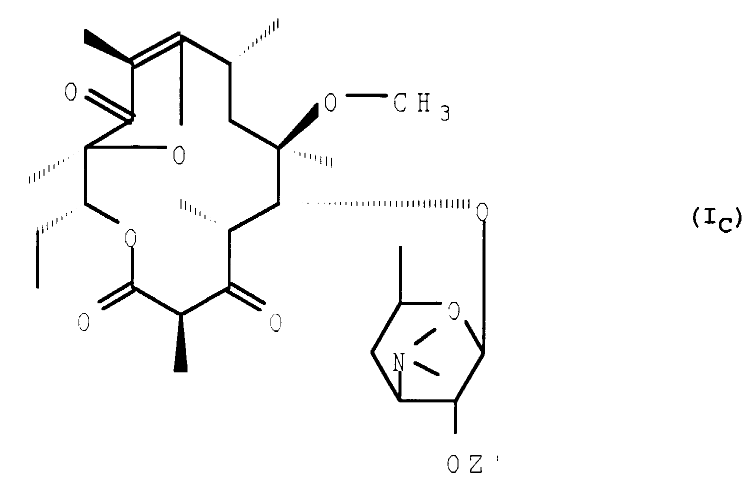
puis soumet si désiré le composé de formule (I_{C}) à l'action d'un agent de libération de la fonction hydroxyle en 2' pour obtenir le composé de formule (I_{D}) :

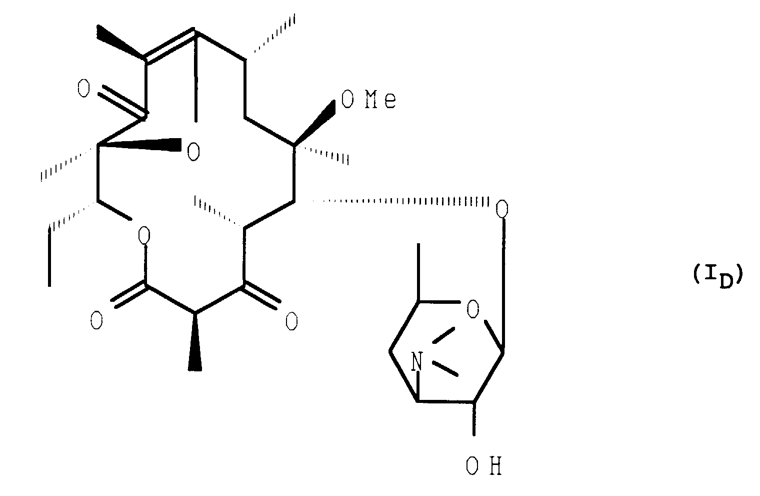

Les composés de formule (II) utilisés comme produits de départ du procédé de l'invention sont décrits et revendiqués dans la demande de brevet européen 487411.

Dans un mode de réalisation préféré de l'invention :
- la réaction d'oxydation en 11 et d'obtention simultanée du pont époxyde en 9(12) est réalisée au moyen du chlorure d'oxalyle dans le diméthylsulfoxyde en présence d'une base de préférence la triéthylamine,
- la libération de la fonction hydroxyle en 2' est effectuée par méthanolyse,
- l'estérification est réalisée au moyen d'acide selon les procédés classiques,
- la déshydratation en 9(10) est réalisée au moyen d'acide chlorhydrique, ou de tout autre acide de type fluoro ou perfluoroacétique, sulfurique,
- la réduction de la double liaison en 9(10) est réalisée à l'aide d'hydrure de diisobutylaluminium (DIBAH), ou par hydrogénation catalytique en présence d'un acide tel que défini ci-dessus.

Les exemples suivants illustrent l'invention.

### EXEMPLE 1 : (9S) 3 de(2,6-didéoxy 3-C-méthyl 3-O-méthyl alpha-L-ribohexopyranosyl) oxy) 9-déoxo 11,12-didéoxy 3,11-dioxo 9,12-époxy 9-hydroxy 6-O-méthyl érythromycine.

### STADE A : 2'-acétate du (9S) 3 de(2,6-didéoxy 3-C-méthyl 3-O-méthyl alpha-L-ribohexopyranosyl) oxy) 9-déoxo 11,12-didéoxy 3,11-dioxo 9,12-époxy 9-hydroxy 6-O-méthyl érythromycine

On refroidit à -70°C, 2,8 cm³ de chlorure d'oxalyle en solution dans 30 ml de chlorure de méthylène. On ajoute 4 cm³ de diméthyl sulfoxyde en solution dans 3 cm³ de chlorure de méthylène. On agite quelques minutes à -70°C et introduit 2 g de 2'-acétate de 3-dé[2,6-didéoxy 3-C-méthyl 3-O-méthyl alpha-L-ribohexopyranosyl) oxy] 6-O-méthyl 3-oxo érythromycine préparé comme indiqué dans le brevet européen 487411 exemple 3C 1), en solution dans 10 cm³ de chlorure de méthylène.

On agite pendant 65 minutes à -70°C et ajoute en 2 h, 20 ml de triéthylamine. On laisse la température remonter à la température ambiante et agite une heure à cette température. On dilue au chlorure de méthylène, lave la phase organique, sèche, filtre et concentre à sec. On obtient 2,06 g de produit que l'on purifie par chromatographie en éluant avec le mélange chlorure de méthylène-méthanol-ammoniaque (98-2-0,2). On obtient ainsi 575 mg de produit recherché.
Spectre IR, CHCl₃
- absence 9 céto
- OH 3578 cm⁻¹
- C=O 1750-1712 cm⁻¹
- bandes de Bohlman -N=
RMN CDCl₃ ppm
H₁₀ 2,55-2,75 ppm

### STADE B : (9S) 3-de(2,6-didéoxy 3-C-méthyl 3-O-méthyl alpha-L-ribohexopyranosyl) oxy) 9-déoxo 11,12-didéoxy 3,11-dioxo 9,12-époxy 9-hydroxy 6-O-méthyl érythromycine

On maintient sous agitation pendant 3 jours à la température ambiante une solution renfermant 100 mg de produit préparé au stade A et 1 cm³ de méthanol. On obtient 100 mg de produit que l'on purifie par chromatographie en éluant avec le mélange acétate d'éthyle-triéthylamine (95-5).

On obtient ainsi 73 mg de produit recherché.
RMN CDCl₃
2,29 (s) : N-(CH₃)₂ ; 4,37 (d) : H'₁ ; 3,20 (dd) : H'₂ ; 2,50 (dd) : H'₃ ; 3,51 (dd) : H'₅.

### EXEMPLE 2 : 3-de((2,6-didéoxy 3-C-méthyl 3-O-méthyl alpha-L-ribohexopyranosyl) oxy) 9-deoxo 9,10-didéhydro 11,12-didéoxy-3,11-dioxo 9,12-époxy 6-O-méthyl érythromycine

### STADE A : 2'-acétate (9S) 3-de((2,6-didéoxy 3-C-méthyl 3-O-méthyl alpha-L-ribohexopyranosyl) oxy) 9-deoxo 9,10-didéhydro 11,12-didéoxy-3,11-dioxo 9,12-époxy 6-O-méthyl érythromycine

On ajoute 8,5 cm³ d'une solution d'acide chlorhydrique 2N dans une solution renfermant 1,28 g de produit préparé à l'exemple 1 stade A et 20 cm³ d'éthanol. On maintient le mélange réactionnel sous agitation pendant 16 heures. On concentre à sec, amène à pH 7-8 par addition d'une solution aqueuse de bicarbonate de sodium, extrait au chlorure de méthylène, sèche, filtre et concentre à sec. On obtient 1,1 g de produit recherché que l'on purifie par chromatographie sur silice en éluant avec le mélange chlorure de méthylène-méthanol-ammoniaque (98-2-0,2). On obtient ainsi 575 mg de produit recherché.
RMN CDCl₃
Absence de proton en 10

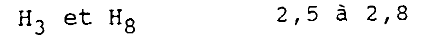

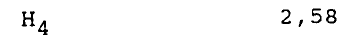

### STADE B : 3-de((2,6-didéoxy 3-C-méthyl 3-O-méthyl alpha-L-ribohexopyranosyl) oxy) 9-deoxo 9,10-didéhydro 11,12-didéoxy-3,11-dioxo 9,12-époxy 6-O-méthyl érythromycine

On agite pendant une nuit à la température ambiante un mélange renfermant 50 mg de produit préparé au stade A de l'exemple 2 et 2 ml de méthanol. On agite la solution pendant une nuit à la température ambiante. On évapore à sec et obtient 40 mg de produit recherché brut que l'on purifie par chromatographie sur silice en éluant avec le mélange acétate d'éthyle-triéthylamine (95-5). On obtient ainsi 45,1 mg de produit recherché.

| RMN CDCl₃ ppm | |
|---|---|
| H'₁ | 4,30 (d) |
| H'₂ | 3,18 (dd) |
| H'₃ | 2,45 (m) |
| H'₅ | 3,51 (m) |

| 227 (J) N (Me)₂ | | |
|---|---|---|
| Analyse : | Calculé | trouvé |
| C = | 63,40 % | 63,1 % |
| H = | 8,7 % | 8,8 % |
| N = | 2,46 % | 2,3 % |

### EXEMPLE 3 : 3-de((2,6-didéoxy 3-C-méthyl 3-O-méthyl alpha-L-ribohexopyranosyl) oxy) 9-deoxo 11,12-didéoxy-3,11-dioxo 9,12-époxy 6-O-méthyl érythromycine

Le produit a été préparé par réduction du composé obtenu au stade A de l'exemple 2, puis en libérant l'hydroxyle en 2' par méthanolyse.

### EXEMPLES de compositions pharmacologiques

On a préparé des comprimés renfermant :

| | |
|---|---|
| Produit de l'exemple 2 | 150 mg |
| Excipient q.s.p. | 1 mg |
| Détail de l'excipient : amidon, talc, stéarate de magnésium. | |

### ETUDE PHARMACOLOGIQUE DES PRODUITS DE L'INVENTION

### A) Activité in vitro.

### Méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats obtenus avec le produit de l'exemple 2 sont les suivants : lecture après 24 H.

| | |
|---|---|
| Streptococcus pyogenes groupe A 02A1UC1 | 1,2 |
| Streptococcus agalactiae groupe B02B1HT1 | 0,15 |
| Streptococcus faecalis groupe D 02D2UC1 | 1,2 |
| Streptococcus faecium groupe D 02D3HT1 | 1,2 |
| Streptococcus groupe G 02G0GR5 | 0,6 |
| Streptococcus mitis 02mitCB1 | 0,6 |
| Streptococcus pneumoniae 032UC1 | 0,3 |
| Streptococcus pneumoniae 030GR20 | 0,15 |

Conclusion : le produit de l'exemple 2 présente une activité antibiotique intéressante.

## Revendications

1. Les composés de formule (I) : 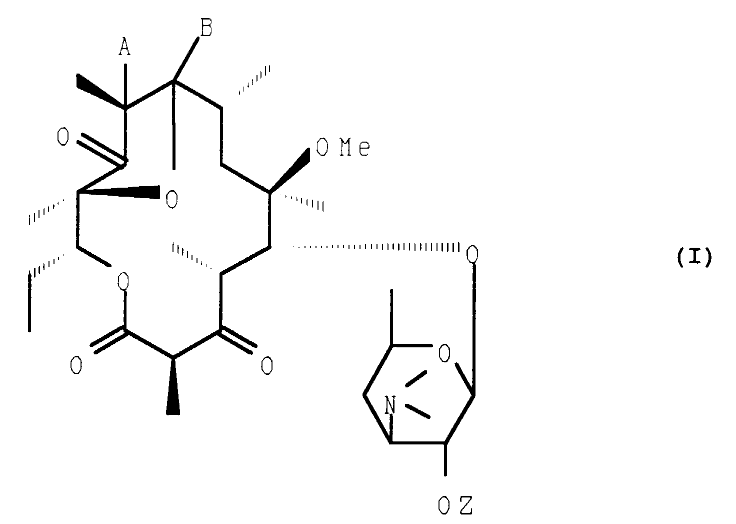 dans lesquels :
- ou bien A représente un atome d'hydrogène et B un radical OH,
- ou bien A et B forment ensemble une double liaison carbone-carbone,
et Z représente un atome d'hydrogène ou le reste d'un acide carboxylique.

2. Les composés de formule (I) tels que définis à la revendication 1 dans lesquels Z représente un atome d'hydrogène.

3. Les composés de formule (I) tels que définis à la revendication 1 ou 2 dans lesquels A et B forment ensemble une double liaison carbone-carbone.

4. 3-de((2,6-didéoxy 3-C-méthyl 3-O-méthyl alpha-L-ribohexopyranosyl) oxy) 9-deoxo 9,10-didéhydro 11,12-didéoxy-3,11-dioxo 9,12-époxy 6-O-méthyl érythromycine.

5. Procédé de préparation des composés de formule (I), caractérisé en ce que l'on soumet un composé de formule (II) : 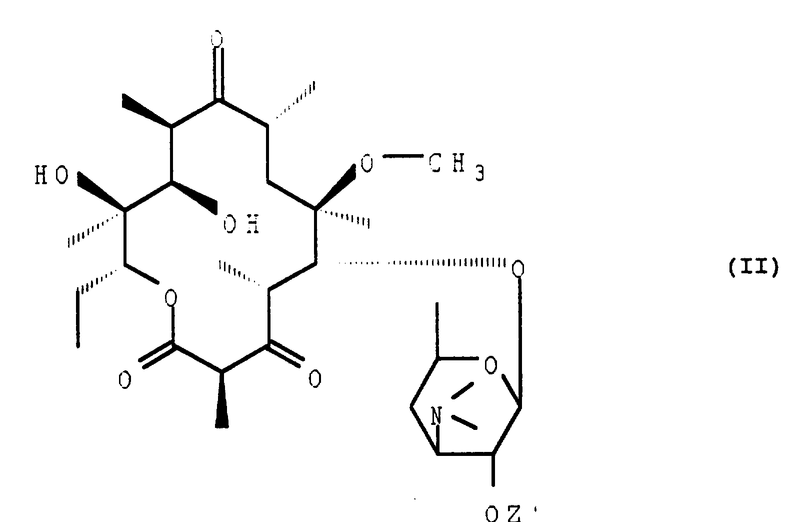 dans laquelle Z' représente le reste d'un acide carboxylique à l'action d'un agent capable d'introduire un pont époxyde en 9(12) et d'oxyder simultanément la fonction hydroxy en 11, pour obtenir le composé de formule (I_{A}) : 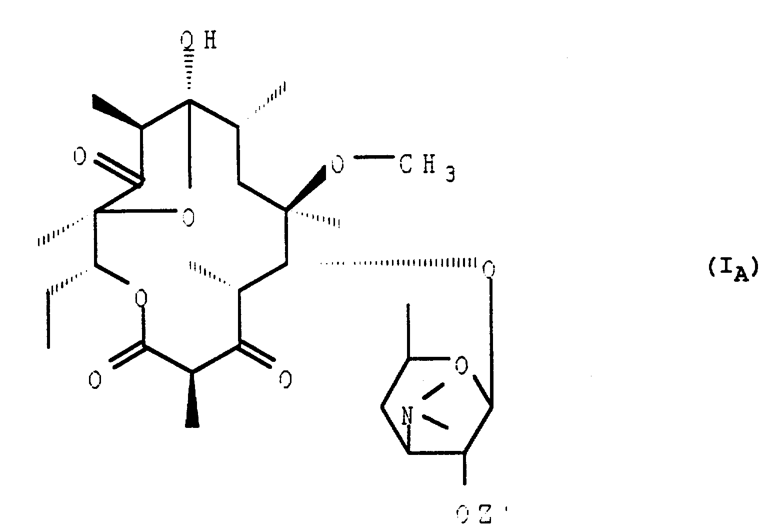 que l'on soumet si désiré :
- soit à l'action d'un agent de libération de la fonction hydroxyle en 2' pour obtenir le composé (I_{B}) : 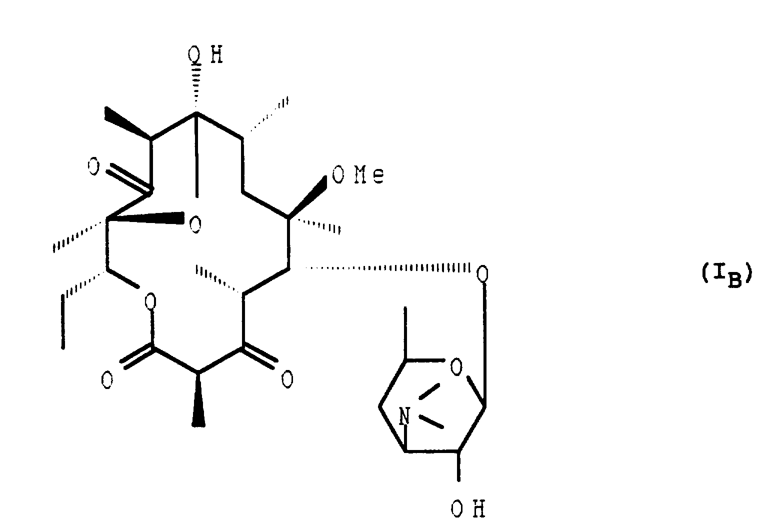
- soit à l'action d'un agent de déshydratation pour obtenir le composé de formule (I_{C}) : 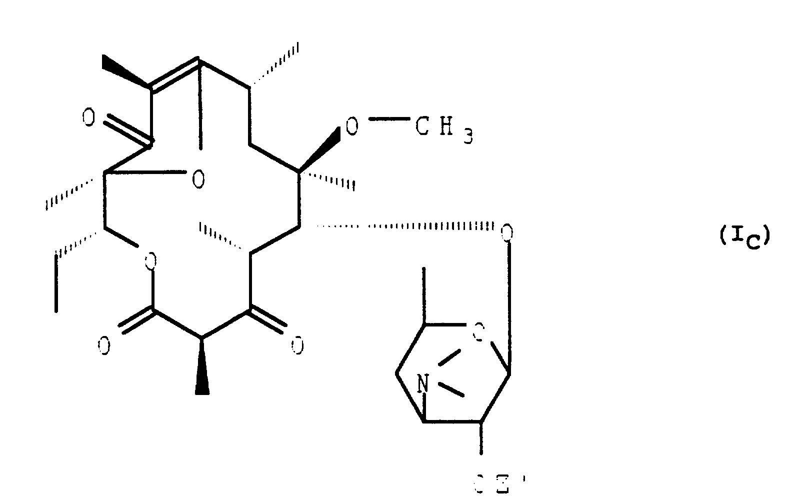
puis soumet si désiré le composé de formule (I_{C}) à l'action d'un agent de libération de la fonction hydroxyle en 2' pour obtenir le composé de formule (I_{D}) : 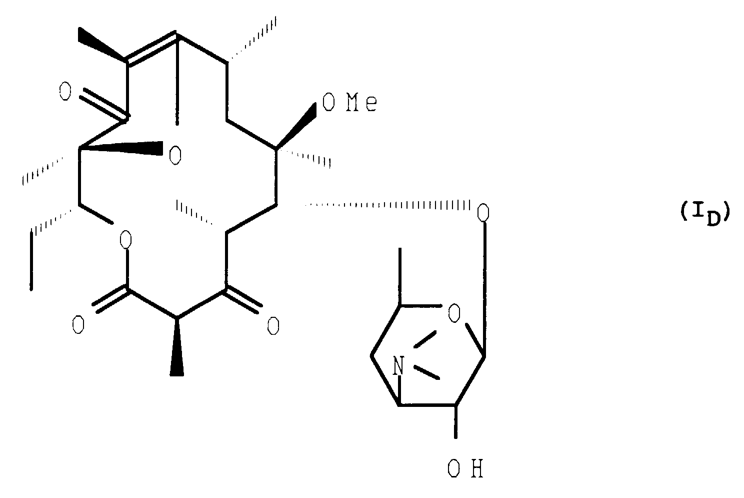 que l'on soumet si désiré à l'action d'un agent de libération de la fonction hydroxyle en 2' pour obtenir le composé de formule (I_{F}) :

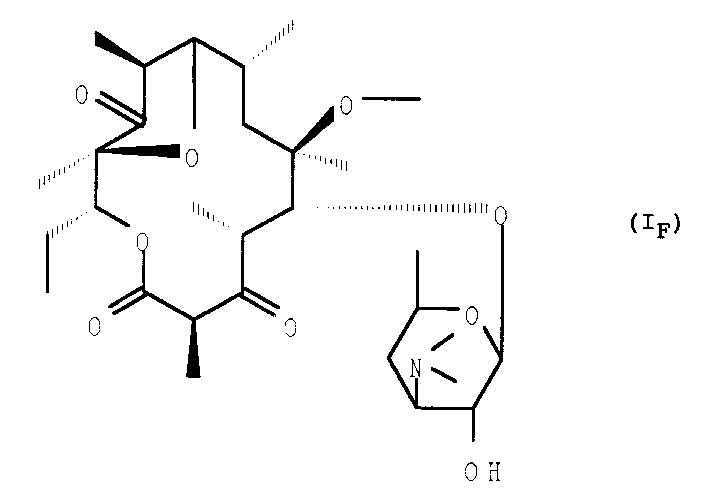

6. A titre de médicaments, les composés de formule (I) tels que définis à l'une quelconque des revendications 1 à 3.

7. A titre de médicament le composé de la revendication 4.

8. Les compositions pharmaceutiques renfermant comme principe actif au moins un médicament défini à la revendication 6 ou 7.

## Patentansprüche

1. Verbindungen der Formel (I) 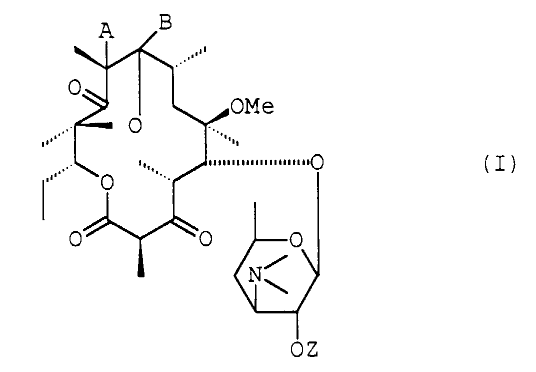 in der
- entweder A ein Wasserstoffatom und B einen Rest OH bedeuten,
- oder A und B zusammen eine Kohlenstoff-Kohlenstoff-Doppelbindung darstellen,
und Z ein Wasserstoffatom oder der Rest einer Carbonsäure ist.

2. Verbindungen der Formel (I) wie in Anspruch 1 definiert, worin Z ein Wasserstoffatom darstellt.

3. Verbindungen der Formel (I) wie in Anspruch 1 oder 2 definiert, worin A und B eine Kohlenstoff-Kohlenstoff-Doppelbindung darstellen.

4. 3-De-[(2,6-dideoxy-3-C-methyl-3-O-methyl-alpha-L-ribohexopyranosyl)-oxy]-9-deoxo-9,10-didehydro-11,12-dideoxy-3,11-dioxo-9,12-epoxy-6-O-methyl-erythromycin.

5. Verfahren zur Herstellung der Verbindungen der Formel (I), dadurch gekennzeichnet, daß man eine Verbindung der Formel (II) 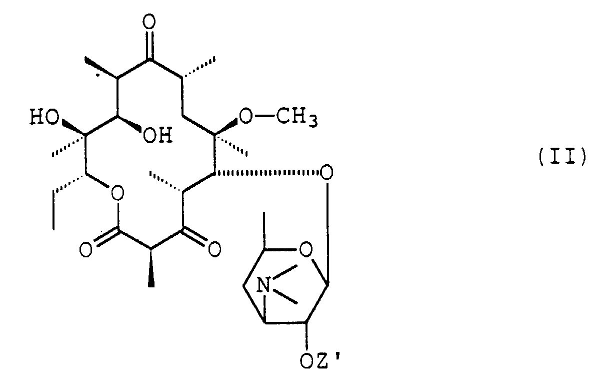 in der Z' den Rest einer Carbonsäure darstellt, der Einwirkung eines Mittels unterzieht, das fähig ist, eine Epoxy-Brücke in 9(12) einzuführen und gleichzeitig die Hydroxyfunktion in 11 zu oxidieren, um die Verbindung der Formel (I_{A}) 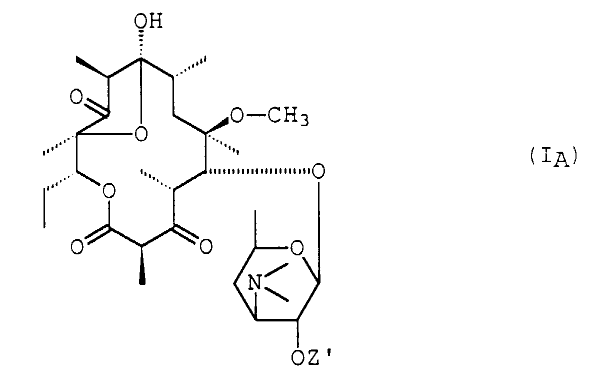 zu erhalten, die man, wenn gewünscht, unterzieht:
- entweder der Einwirkung eines Mittels zur Freisetzung der Hydroxylfunktion in 2', um die Verbindung der Formel (I_{B}) 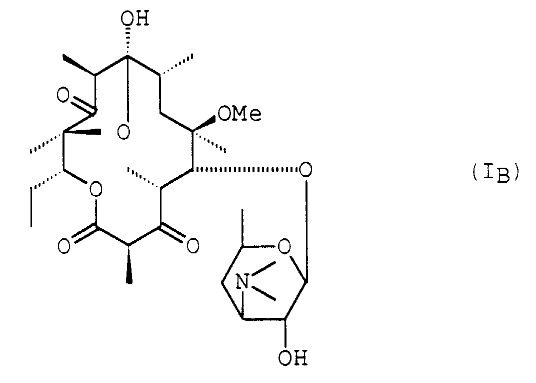 zu erhalten, oder
- der Einwirkung eines Mittels zur Dehydratisierung, um die Verbindung der Formel (I_{C}) 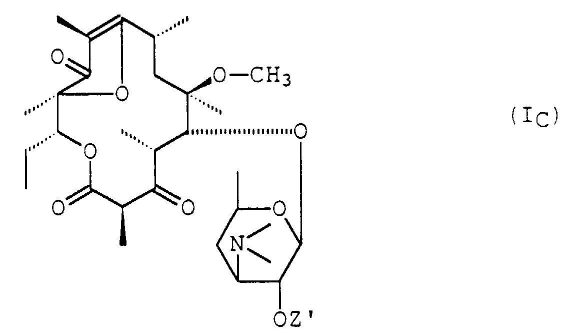
zu erhalten, und man anschließend, wenn gewünscht, die Verbindung der Formel (I_{C}) der Einwirkung eines Mittels zur Freisetzung der Hydroxylfunktion in 2' unterzieht, um die Verbindung der Formel (I_{D}) 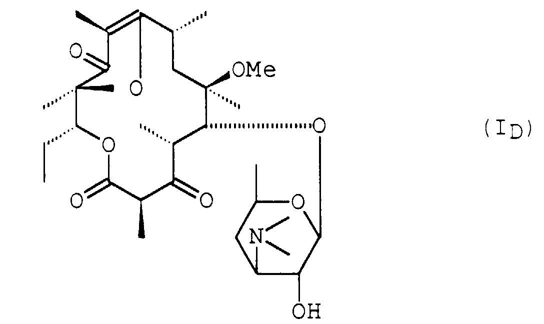 zu erhalten, die man, wenn gewünscht, der Einwirkung eines Mittels zur Freisetzung der Hydroxylfunktion in 2' unterzieht, um die Verbindung der Formel (I_{F}) 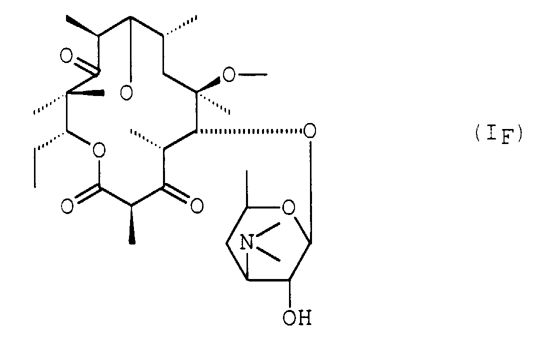 zu erhalten.

6. Als Medikamente die Verbindungen der Formel (I) wie in irgendeinem der Ansprüche 1 bis 3 definiert.

7. Als Medikament die Verbindung von Anspruch 4.

8. Pharmazeutische Zusammensetzungen, die als Wirkstoff mindestens ein Medikament enthalten wie in Anspruch 6 oder 7 definiert.

## Claims

1. The compounds of formula (I): 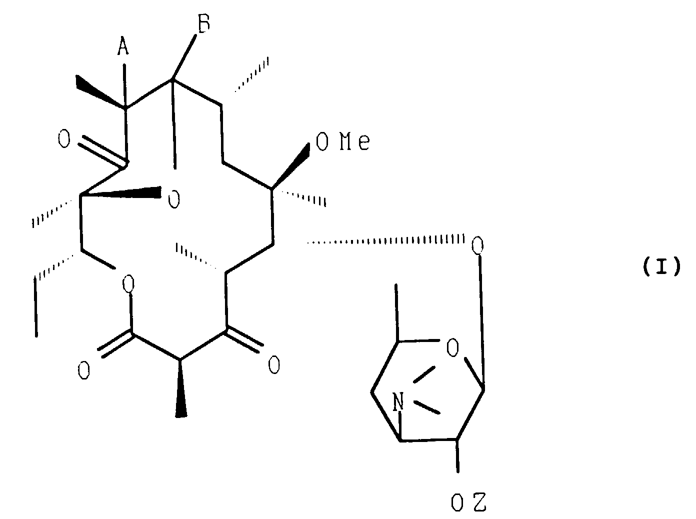 in which:
- either A represents a hydrogen atom and B an OH radical,
- or A and B form together a carbon-carbon double bond,
and Z represents a hydrogen atom or the remainder of a carboxylic acid.

2. The compounds of formula (I) as defined in claim 1 in which Z represents a hydrogen atom.

3. The compounds of formula (I) as defined in claim 1 or 2 in which A and B form together a carbon-carbon double bond.

4. 3-de((2,6-dideoxy 3-C-methyl 3-O-methyl alpha-L-ribohexopyranosyl) oxy) 9-deoxo 9,10-didehydro 11,12-dideoxy-3,11-dioxo 9,12-epoxy 6-O-methyl erythromycin.

5. Preparation process for the compounds of formula (I), characterized in that a compound of formula (II): 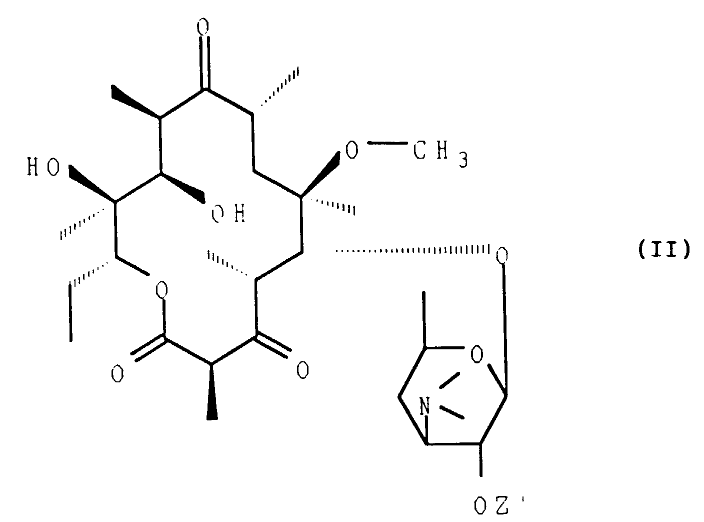 in which Z' represents the remainder of a carboxylic acid, is subjected to the action of an agent capable of introducing an epoxide bridge in position 9(12) and of simultaneously oxidizing the hydroxy function in position 11, in order to obtain the compound of formula (I_{A}): 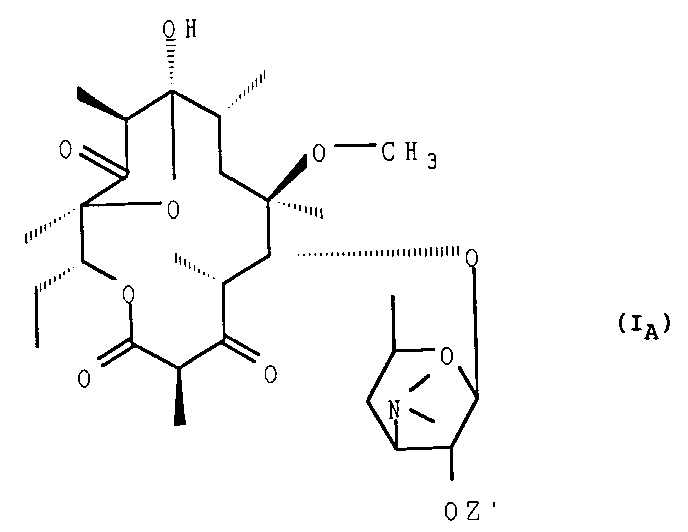 which is subjected if desired:
- either to the action of a releasing agent of the hydroxyl function in position 2' in order to obtain the compound (I_{B}): 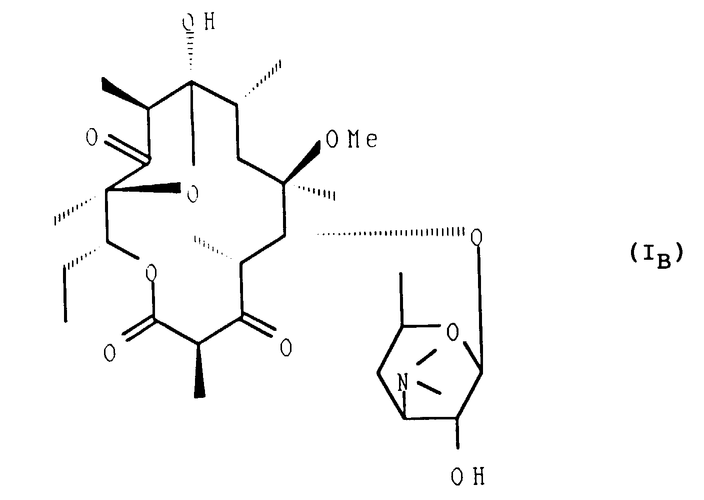
- or to the action of a dehydration agent in order to obtain the compound of formula (I_{C}): 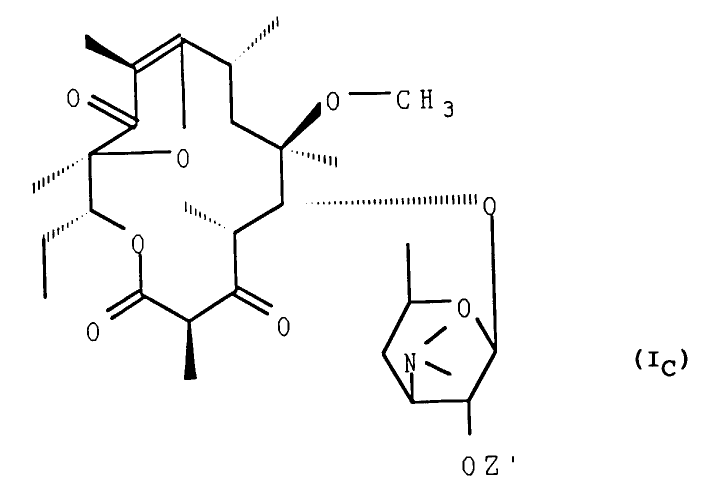
then if desired the compound of formula (I_{C}) is subjected to the action of a releasing agent of the hydroxyl function in position 2' in order to obtain the compound of formula (I_{D}): 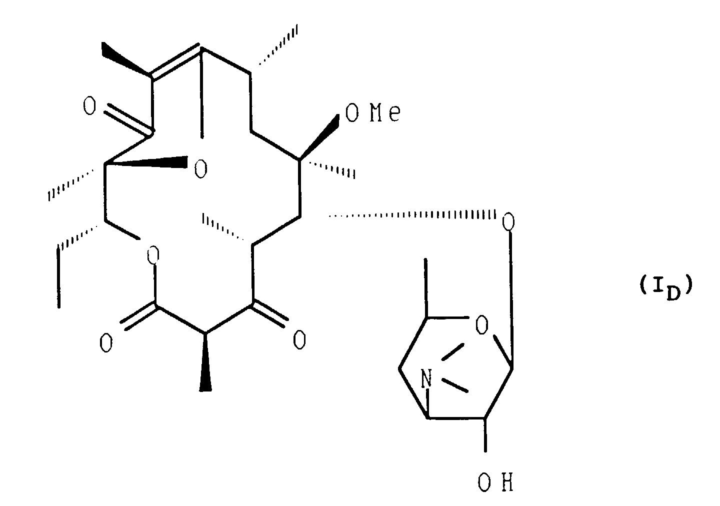 which is subjected if desired to the action of a releasing agent of the hydroxyl function in position 2' in order to obtain the compound of formula (I_{F})):

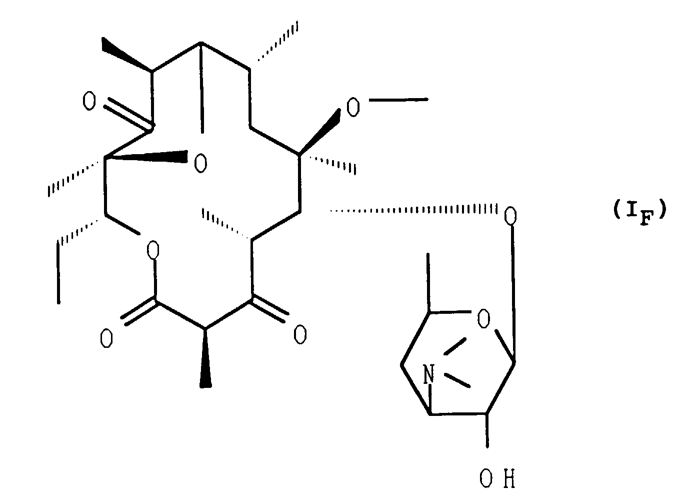

6. As medicaments, the compounds of formula (I) as defined in any one of claims 1 to 3.

7. As a medicament, the compound of claim 4.

8. The pharmaceutical compositions containing as active ingredient at least one medicament defined in claim 6 or 7.
